# EUROPÄISCHE PATENTANMELDUNG

(11) **EP 3 827 870 A1**
(43) Veröffentlichungstag der Anmeldung: **02.06.2021**
(21) Anmeldenummer: 20210168.9
(22) Anmeldetag: 27.11.2020
(51) Int. Cl.: A61M 37/00, A61K 9/00, A61N 2/00

(54) **IMMUNMODULATORANORDNUNG ZUR BEHANDLUNG VON KREBSERKRANKUNGEN**

(30) Priorität: 29.11.2019 DE 102019132549
(71) Anmelder: FRAUNHOFER-GESELLSCHAFT zur Förderung der angewandten Forschung e.V., 80686 München (DE)
(72) Erfinder: Fabian, Dr. Claire, 04103 Leipzig (DE); Fricke, Dr. Stephan, 04103 Leipzig (DE); Lausch, Dr. Holger, 07629 Hermsdorf (DE); Brand, Michael, 07629 Hermsdorf (DE)
(74) Vertreter: Gleim Petri Oehmke Patent- und Rechtsanwaltspartnerschaft mbB

(57) **Zusammenfassung**

Der Erfindung liegt die Aufgabe zugrunde, eine effektive Möglichkeit zur Mobilisierung des körpereigenen Immunsystems im Kampf gegen Krebs vorzuschlagen.

Die Aufgabe wird gelöst durch eine Immunmodulatoranordnung, bestehend aus einem immunmodulatorischen *in-vivo-*Implantat (1) mit einer Endo-/Laparoskopiehalterung (2) und einem Ex-vivo-Wechselfeldgenerator, wobei das immunmodulatorische *in-vivo-*Implantat (1) als mechanotransduktiv wirkendes elektromagnetisches Induktor- und akustisches Wandlersystem ausgebildet ist und / oder das immunmodulatorische *in-vivo-*Implantat (1) signalgebende Oberflächenmerkmale aufweist, so dass nach einer endoskopischen/laparoskopischen Platzierung am oder im Tumorgewebe oder am Transplantat eine mechanotransduktive Immunmodulation und / oder eine aktive und / oder passive Applikation von ein-/angelagerten Signalwirkstoffen zur signaltransduktiven, immunmodulatorischen Therapie durchführbar ist.

## Beschreibung

Die Erfindung betrifft eine Immunmodulatoranordnung und ein Verfahren zur Mobilisierung des körpereigenen Immunsystems im Kampf gegen Krebs, insbesondere gegen das Adenokarzinom des Pankreas. Die Immunmodulatoranordnung ist ebenfalls geeignet, eine Immunsuppression positiv zu beeinflussen.

Im Internet ist unter https://www.onkopedia.com/de/onkopedia/guidelines/pankreaskarzi nom/@@guideline/html/index.html (Stand Oktober 2018) ein Artikel veröffentlicht, der sich ausgiebig mit dem Adenokarzinom des Pankreas beschäftigt, aus dem nachfolgend einzelne Passagen auch teilweise wörtlich übernommen wurden.
Das Adenokarzinom des Pankreas steht bei Frauen an sechster, bei Männern an zehnter Stelle der häufigsten, neuaufgetretenen Krebserkrankungen. Das mittlere Erkrankungsalter liegt zwischen 70-75 Jahren, Personen mit genetischer oder erworbener Belastung können schon im frühen Erwachsenenalter erkranken. Etwa 70 % der Karzinome sind im Pankreaskopf lokalisiert. In Deutschland werden jährlich ungefähr 8.800 Neuerkrankungen bei Männern und ca. 8.300 bei Frauen diagnostiziert. Die absolute 5-Jahres-Überlebensrate wird mit 8 % (Männer) bzw. 9 % (Frauen) angegeben. Es zeigte sich zudem in den letzten 10 Jahren ein jährlicher Anstieg der altersbereinigten Inzidenzraten um 2,1 %. Bei diesem statistisch signifikanten Anstieg handelt es sich um eine tatsächliche Zunahme der Erkrankungs- und Sterbehäufigkeit. Zum Zeitpunkt der Erstdiagnose wird eine resektable Erkrankung nur bei einer Minderheit der Patienten (15-20 %) gefunden.
Über bildgebende Verfahren ist der Zustand der Bauspeicheldrüse nur bedingt zu beurteilen, da die Lage zwischen Lungen, Magen und Gallenblase sowie die Rippenstrukturen z. B. mit Ultraschall sehr häufig nur mit Artefakten zu sehen ist. Ein CT/MRT sowie Endosonografie sind in diesem Falle geeignetere bildgebende Verfahren, um die Ausbreitung des Tumors zu evaluieren. Die Bestimmung der Tumormarker CA 19-9 und CEA im peripheren Blut sind ergänzend empfohlen.
Es gibt vielversprechende Ansätze für die Therapie von Pankreaskarzinomen mit CAR-T-Zellen, die aber Technologien benötigen, um die antigengerichtete Aktivität zu verbessern und die Toxizität der Behandlung zu verringern [Front Immunol. 2018; 9: 2166. Published online 2018 Sep 25. doi: 10.3389/fimmu.2018.02166]. Auch NK-CAR-Zellen können eine zukünftige therapeutische Option darstellen [Pharmacol Ther. 2018 Sep; 189:31-44. doi: 10.1016/j.pharmthera.2018.04.003. Epub 2018 Apr 13]. Problematisch ist die Fähigkeit dieser Immunzelltherapeutika, die Matrix des Pankreaskarzinoms zu durchdringen und lokal, das heißt in Nähe der Tumorzellen, aktiv werden zu können.
Das Immunsystem und ebenso immunonkologische Zelltherapeutika (ATMPs) können über mechanische Reize beeinflusst werden.
Makrophagen können über zelluläre Mechanosensoren aktiviert werden. Unter anderem verfügen sie über spezialisierte Zell-Matrix-Adhäsionsbereiche (Podosomen), die auf mechanotransduktive Signale reagieren können. Alleine unterschiedlicher mechanischer Stress (Zug) kann z. B. die Makrophagendifferenzierung in Scaffolds modulieren [Ballotta V, Driessen-Mol A, Bouten CVC, Baaijens FPT. Strain-dependent modulation of macrophage polarization within Scaffolds. Biomaterials. 2014;35:4919-28. doi: 10.1016/j.biomaterials.2014.03.002].
Dabei sind die Effekte abhängig von der Stärke des mechanischen Stresses. Es können sowohl pro-, wie anti-inflammatorische Differenzierungen ausgelöst werden. Es kann aber auch bei zu hohem Stress zum Zelltod kommen. Obgleich bereits bekannt ist, dass mechanotransduktive Prozesse sowohl Inflammations- als auch Regenerationsprozesse maßgeblich beeinflussen, gibt es bisher keine Möglichkeit einer gezielten topischen Stimulation der mechanotransduktiven Prozesse mit definier- und messbaren mechanischen Stimuli (Frequenz, Amplitude, Signalform, Signaldauer).

Der Erfindung liegt die Aufgabe zugrunde, eine effektive Möglichkeit zur Mobilisierung des körpereigenen Immunsystems im Kampf gegen Krebs vorzuschlagen.

Die Aufgabe wird gelöst durch eine Immunmodulatoranordnung, bestehend aus einem immunmodulatorischen *in-vivo*-Implantat mit einer Endo-/Laparoskopiehalterung und einem Ex-vivo-Wechselfeldgenerator, wobei das immunmodulatorische *in-vivo-*Implantat als mechanotransduktiv wirkendes elektromagnetisches Induktor- und akustisches Wandlersystem ausgebildet ist und / oder das immunmodulatorische *in-vivo*-Implantat signalgebende Oberflächenmerkmale aufweist, so dass nach einer endoskopischen/laparoskopischen Platzierung am oder im Tumorgewebe oder am Transplantat eine mechanotransduktive Immunmodulation und / oder eine aktive und / oder passive Applikation von ein-/angelagerten Signalwirkstoffen zur signaltransduktiven, immunmodulatorischen Therapie durchführbar ist.
Der wesentliche Vorteil der erfindungsgemäßen Immunmodulatoranordnung besteht darin, dass ein duales Verfahren auf der Basis einer gemeinsamen technischen Plattform, dem in-vivo-Implantat, durchgeführt werden kann. Dabei kommen zwei Ansätze zum Tragen:
Mechanotransduktive proinflammatorische Immunmodulation über ein minimalinvasiv endoskopisch/laparoskopisch temporär implantierbares *in-vivo*-Implantat, welches als Vibrationskörper mit einer speziellen Oberfläche ausgebildet ist, für eine signaltransduktive, immunmodulatorische Therapie bzw. aktive und / oder passive Applikation von Signalwirkstoffen gegen solide Tumore über die Oberfläche.
Dies ermöglicht die gezielte topische Immunmodulation durch die Anregung endogener zellulärer Prozesse über Mechanotransduktion und / oder durch die exogene Induktion über Signalwirkstoffe. Diese mechanotransduktive und signaltransduktiv-onkologische Immunmodulatoranordnung basiert auf einem magnetischen Energieübertragungsverfahren von einem Ex-vivo-Wechselfeldgenerator an ein *in-vivo-*Implantat, welches über endoskopische oder laparoskopische Instrumente topisch platzierbar ist.
Es entsteht erstmals die Möglichkeit, systemisch schwer erreichbare bzw. maskierte Karzinome direkt zu erreichen. Topisch genau können Immunzellen immunmodulatorisch beeinflusst werden. Da das modulatorische, elektromagnetische Anregungsprinzip (elektromagnetische Welle) und das monitorende akustische Signal (mechanische Welle) sich als voneinander unabhängig wirkende Verfahren nicht gegenseitig beeinflussen können, bestehen sehr gute systemische Voraussetzungen für die therapeutisch begrenzte Wirkung der Immunmodulation im bzw. am Tumor selbst. Der transitorische Weg vom Ex-vivo- zum *in-vivo*-Tumorraum ist dabei effekt- und damit nebenwirkungsfrei. Auch ein paralleles ultraschallbasiertes Monitoring als Therapievalidierung kann durch den Aktor nicht beeinflusst oder verfälscht werden, da er als lokale Quelle erkennbar ist und die Frequenz zum eventuell notwendigen Ausfiltern bekannt ist.
Über die definierten und strukturierbaren Oberflächen können Wirkstoffe und -signale topisch an bzw. in den Tumor gebracht werden und ermöglichen so eine kontrollierte topische Freisetzung.
Durch die lokal begrenzte topische Applikation von mechanotransduktiven Signalen und / oder Applikation von Wirkstoffen wird die Belastung des Körpers im Vergleich zu einer systemischen Applikation reduziert. Es kann für die Immunmodulation keine transitorische Beeinflussung durch eine Ex-vivo-Ultraschallquelle am Karzinom als potentielle Nebenwirkung erfolgen, da die Mechanotransduktion lokal vor Ort erzeugt wird. Die Leistung ist im Zielgebiet am höchsten und nimmt über die dazwischenliegenden Bereiche radial ab. Die transitorischen Verfahren haben dagegen am Zielort der Applikation in der Regel die geringere Leistung, und ihre höchste am primären Transitkontaktbereich, der naturgemäß an der Hautoberfläche liegt. Eine rein endoskopische Lösung würde eine permanente minimalinvasive Lösung mit potenziell lokalen Nebenwirkungen und mechanischen Komplikationen sowie der Notwendigkeit repetitiver endoskopischer Eingriffe sowie ein höheres Infektionsrisiko durch das permanente Vorhandensein des Vibrationskörpers bedeuten. Des Weiteren ist über eine Integration von drahtlos aufladbaren Akkumulatoren und einem zeit- und leistungsgetriggerten Betriebsregimes eine zeitweise bis dauerhaft nichtinvasive bzw. nicht ortsgebundene Anwendung zu Hause möglich. Damit kann die Lebensqualität der Anwender bzw. der Patienten deutlich gesteigert werden.
Für die statische und dynamische sowie direkte und indirekte Signaltransduktion von der Oberfläche des *in-vivo*-Implantats zu Bestandteilen des Immunsystems sind verschiedene Anwendungsszenarien denkbar:
- eine direkte mechanotransduktive Signaltransduktion von der Oberfläche zu Immunzellen, Gewebe und / oder Wirkstoffen,
- eine Mechano- und biochemische Signaltransduktion von an der Oberfläche fixierten Wirkstoffen zu Immunzellen, Gewebe und / oder Wirkstoffen,
- eine biochemische Signaltransduktion von an der Oberfläche gebundenen Liganden zu Immunzellen, Gewebe und / oder Wirkstoffen mit oder ohne mechanotransduktive(r) Unterstützung oder Initialisierung der signaltransduktiven Kaskade.
Eine weitere erfindungsgemäße Anwendung ist die Verwendung einer Immunmodulatorkapsel in Gestalt des *in-vivo*-Implantats im alleinig mechanotransduktiven Betrieb. Das Monitoring der Positionierung und des Betriebs des *in-vivo*-Implantats wird mittels klassischem, medizinisch-diagnostischem Ultraschall (MDUS) dargestellt. Da die Parameter, der Zeitpunkt und der Ort der Signalgeneration/- applikation bekannt sind, kann mittels des medizinisch-diagnostischen Ultraschalls (MDUS) auch jede Gewebeveränderung im Bereich zwischen dem *in-vivo*-Implantat und dem MDUS orts- und zeitaufgelöst detektiert werden, da das MDUS sehr genau weiß, welches generierte und applizierte, mechanotransduktive oder ein für optische Vergleichsanalysen geeignetes generiertes und appliziertes Testsignal gesucht werden muß.
Die Parameter des generierten Signals können auch differieren, um die Ortsauflösung von Gewebeveränderungen über geeignete Suchalgorithmen und veränderbare Signaldetektionsparameter des MDUS zu erhöhen bzw. zu verbessern. Das signalgenerierende und -applizierende *in-vivo*-Implantat kann dann bspw. in neuronalonkologischen Anwendungsfällen über starre und oder flexible Endoskope mittels makroskopischer Gewebe- und mikroskopischer Zellanalyse/-diagnostik zur Erkennung und Bewertung von Gewebeveränderungen eingesetzt werden. Zusätzlich können solche Systeme mit drahtlos versorgten, gekapselten und implantierbaren Videoendoskopiesystemen gekoppelt und ergänzt werden.

Durch die Strukturierung und Zusammensetzung der Oberfläche kann eine direkte Interaktion mit dem umliegenden Gewebe und Zellen erfolgen, um z. B. eine Adhäsion zu erleichtern oder zu verhindern. Die Modifizierbarkeit der Oberflächeneigenschaften in Bezug auf den Kontaktwinkel, die Struktur und chemische Zusammensetzung ermöglicht die direkte Anbindung von Wirkstoffen (z. B. über kupferfreie Klickchemie, Beladung in Poren, Einbindung in ECM-Substraten). Diese können direkt vor Ort wirken und / oder passiv über Diffusion oder aktiv mechanotransduktiv freigesetzt werden. Dies ermöglicht eine möglichst topische Darreichung von Wirkstoffen von u.a. Antibody-drugconjugates, Checkpoint-Inhibitoren, Chemotherapeutika, Tumorvakzinen und / oder Zytokintherapeutika.
Die Porosität der Oberfläche des Materials kann so adaptiert werden, dass im Unterschied zum klassischen Drug Delivery Habitate für konventionelle Zell- und Gentherapeutika geschaffen werden können.

In einem solchen Habitat können konventionelle Zelltherapeutika, wie hämatopoetische Stammzellzubereitungen sowie Mesenchymale Stammzellen (MSCs), immunregulatorische Zellen, z.B. regulatorische T-Lymphozyten oder Makrophagen sowie andere Arzneimittel für Neuartige Therapien (*Advanced Therapy Medicinal Products,* ATMPs) eingebracht werden und lokal die gewünschte immunologische Reaktion (z. B. Tumorzellzerstörung, Immunregulation, Freisetzung von Mediatoren, Aktivierung von lokalen Zelltypen, etc.) ermöglichen. Die Zellen können dabei je nach Zelltyp einzeln sowie im 3D-Verbund (z.B. Sphäroide, Organoide) eingebracht werden. Die topische Immunmodulation kann genutzt werden, um im Gewebe und im Tumor vorhandene Makrophagen und T-Zellen sowie andere Immunzellen zu (re-)aktivieren oder zu supprimieren. Dabei kann eine Kombinationstherapie mit neuen Zelltherapeutika (ATMPs) direkt oder indirekt (siehe oben) erfolgen. Je nach Tumorart und Mechanismus zur Immunmaskierung kann dies vor, während und oder nach Applikation der ATMPs erfolgen, um lokal die körpereigene Immunreaktion oder die Zelltherapeutika zu modulieren. Beispiele für in der Immunonkologie eingesetzten ATMPs sind: TCR-engineered T cells, T cell clones, CAR-ATMPs (Maus MV et al. Blood. 2014 Apr 24;123(17):2625-35).
Durch die Modifizierbarkeit der Oberflächeneigenschaften in Bezug auf die Struktur (Poren/Rillen) und physikalischen und chemischen Eigenschaften kann die Oberfläche als topisches Transportsystem genutzt werden. Denkbar wäre eine Beladung mit Zellen in Kombination mit einer Poren-/Rillenstruktur sowie einer schnell resorbierbaren Schutzschicht, um die Zellen vor einem Abrieb bei der Applikation zu schützen und eine optimale Umgebung zu schaffen. Diese Strukturen können auch zur Beladung mit Substrat, Wirkstoffen und Ähnlichem dienen. Die Veränderung des Kontaktwinkels (hydrophil/hydrophob) ermöglicht eine direkte Modifikation der Oberfläche, z. B. durch Klickchemie, und kann auch zur direkten Bindung von Wirkstoffen genutzt werden. Des Weiteren kann die Oberfläche mit Redox-Paaren mit einem unterschiedlichen Standardelektrodenpotential gemäß der elektrochemischen Spannungsreihe ausgestaltet sein. Die daraus resultierenden Gleichfelder können durch von außen angelegte Wechselfelder definiert verändert werden und elektrische Dipolmomente können induziert werden. Je höher die Polarisierbarkeit der Moleküle in einer chemischen Bindung ist, desto leichter kann über das o. g. Verfahren eine relative Verschiebung von positiven Ionen zu negativen Ionen oder eine Verschiebung des Atomkerns zur umgebenden Elektronenwolke provoziert werden, welche die chemischen Bindungen über elektrostatische Wechselwirkungen beeinflusst.
Wählt man zwei parallele Platten, Netze oder andere Scaffolds mit gleichen oder unterschiedlichen Standardpotentialen als Reservoir für z. B. Wachstumsfaktoren, Zytokinen oder therapeutisch optimierte Stammzellen mit extrazellulärer Matrix, induziert man durch eine Änderung der Feldlinien die o. g. Redox-Paare. Durch Anlegen eines externen magnetischen Wechselfeldes werden die Feldlinien sowie die damit verbundenen Feldstärken in Richtung und Betrag verändert. Da die Zellen sich an den Gleichfeldlinien orientieren, werden im Scaffold befindliche Zellen aktiviert und / oder simuliert und über den Einfluss auf Bindungsenergien werden Wirkstoffe freigesetzt.

Nachfolgend soll die Erfindung anhand von Ausführungsbeispielen mithilfe von Zeichnungen näher erläutert werden. Hierzu zeigen:
- Fig. 1: schematische Darstellung eines *in-vivo*-Implantats mit einem Bieger,
- Fig. 2: schematische Darstellung eines *in-vivo*-Implantats mit einem fest verbundenen Endoskop,
- Fig. 3: schematische Darstellung der Oberfläche eines *in-vivo*-Implantats,
- Fig. 4: schematische Darstellung eines *in-vivo*-Implantats mit einem Bieger und einer teilweise elektrisch kontaktierten Oberfläche,
- Fig. 5: schematische Darstellung von gegenübergestellten Ausführungsformen eines *in-vivo*-Implantats,
- Fig. 6: schematische Darstellung eines mittels Endoskop platzierten *in-vivo-*Implantats,
- Fig. 7: schematische Darstellung einer in Funktion befindlichen Immunmodulatoranordnung,
- Fig. 8: schematische Darstellung eines *in-vivo*-Implantats mit Multilayer-Piezoaktoren,
- Fig. 9: schematische Darstellung eines *in-vivo*-Implantats mit einem Rohr-Piezoaktor,
- Fig. 10: totale Zellzahl nach 24 h mechanotransduktivem Stimulus,
- Fig. 11: Änderung der Zellmorphologie nach 24 h mechanotransduktivem Stimulus und
- Fig. 12: Expression von Oberflächenmarkern MHCII, CD80, CD206 und Dectin nach 24 h mechanotransduktivem Stimulus.

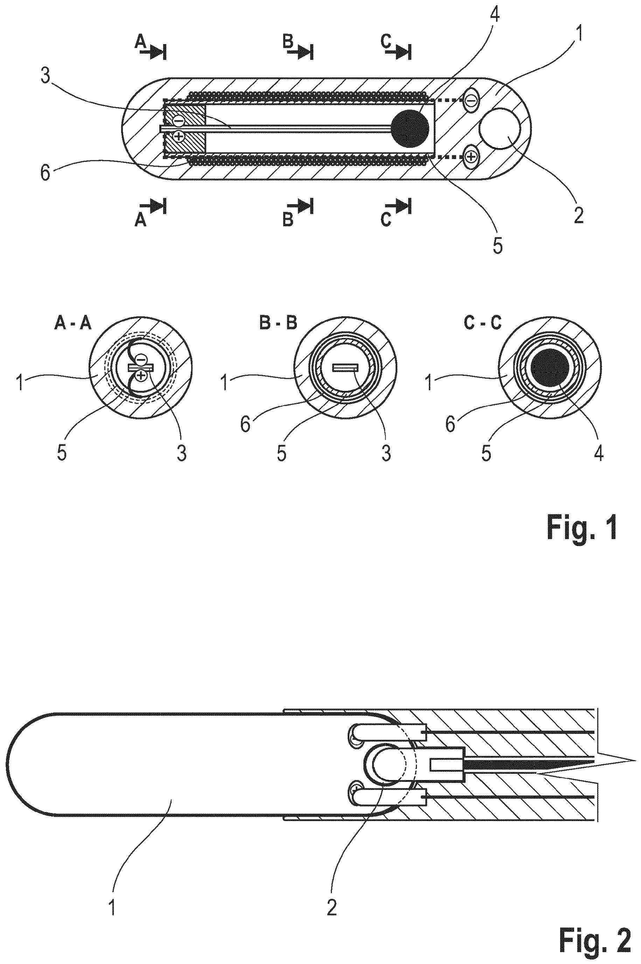

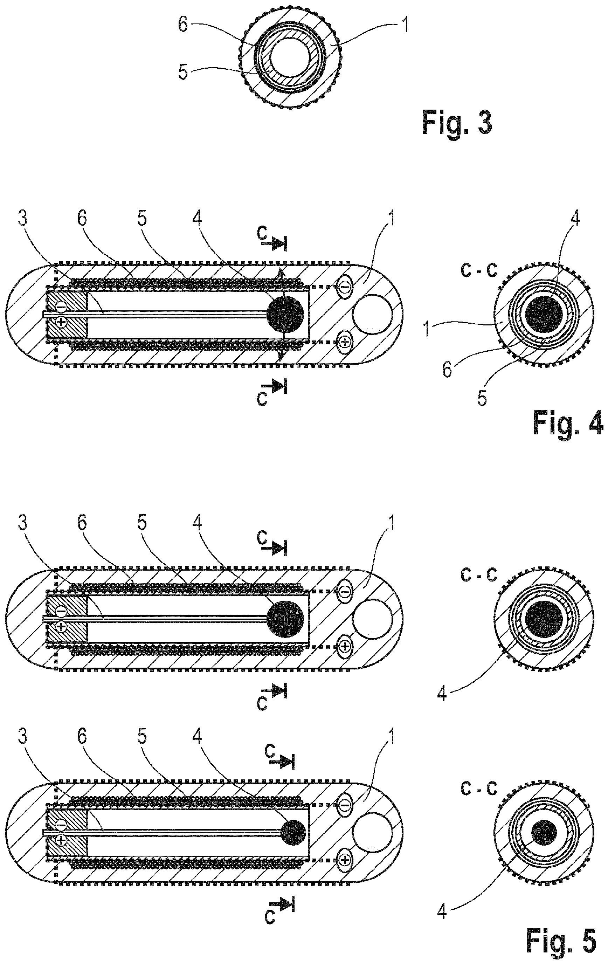

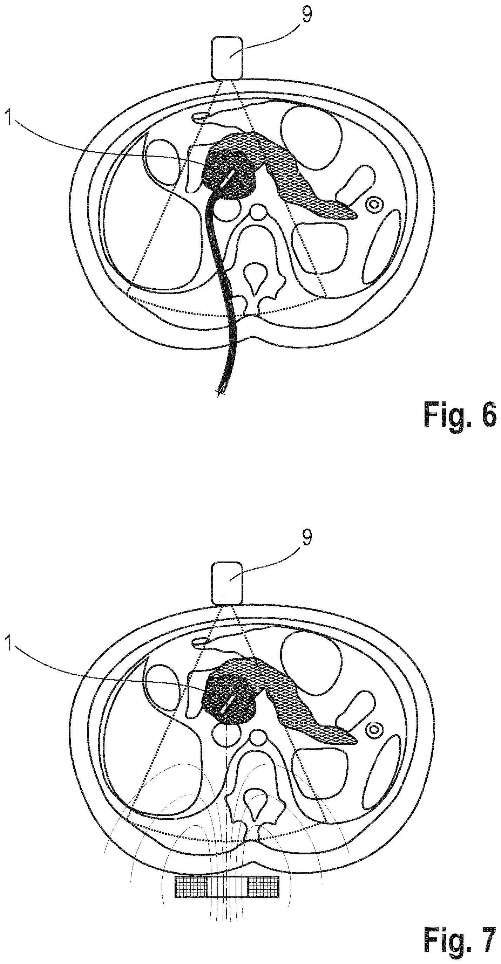

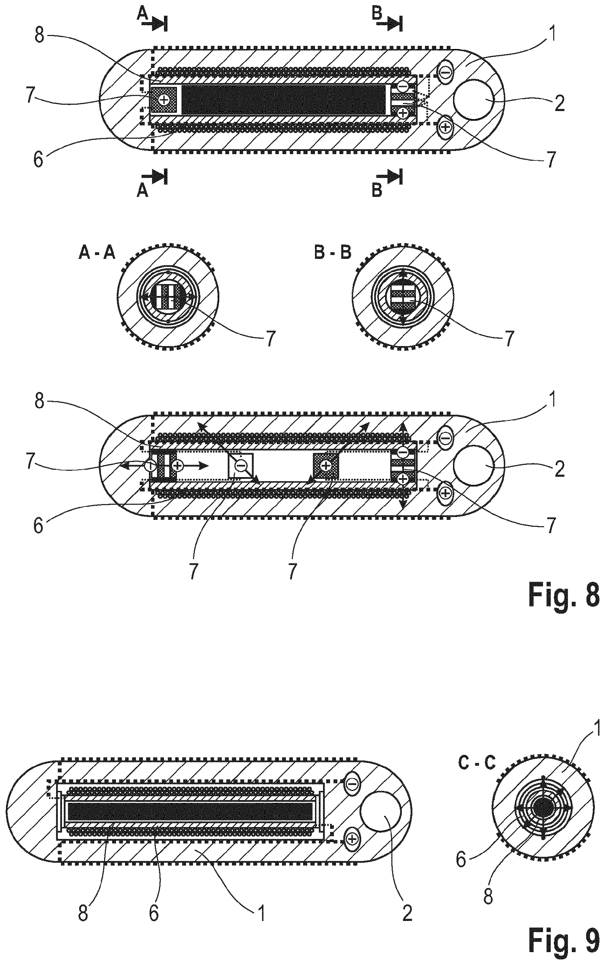

In Fig. 1 ist ein in torpedoähnlicher Form ausgebildetes *in-vivo*-Implantat 1 mit einer Endo-/Laparoskopiehalterung 2 dargestellt. Das *in-vivo*-Implantat 1 kann über die Endo-/Laparoskopiehalterung 2, wie in Fig. 2 dargestellt, mit einem endoskopischen Instrument zur topisch genauen Platzierung an und / oder in einen soliden Tumor temporär fest verbunden werden. Das Monitoring der Platzierung und der nachfolgenden Funktionalität kann über etablierte Ultraschalltechniken erfolgen. Das *in-vivo*-Implantat 1 kann vorteilhaft einerseits aus einem soliden, spanend geformten oder andererseits aus einem 3D-additiv gefertigten Körper bestehen. Das Material dieses Körpers kann dabei aus metallischen, aus Kunststoff-, keramischen und / oder Verbund-/Kompositwerkstoffen bestehen. Dabei entsteht ein radialer Oberflächenbereich mit einer beliebig dreidimensional gestaltbaren Oberflächenform. Diese kann mit dem Ziel der Oberflächenvergrößerung wellen-, stern- und / oder spiralförmig und mit unterschiedlicher Rauhigkeit bis hin zu Porositäten über additive oder abrasive Verfahren gestaltet sein (Fig. 3). An diese strukturierte Oberfläche können direkt oder über spezifische Koppel-/Bindungsbereiche signaltransduktive, materialtechnische oder biochemische Stoffe, Materialien oder Komponenten temporär bzw. dauerhaft angebunden werden.
Die in Fig. 3 angedeutete radiale und Längs-/Quer-Führungs- und Oberflächenstruktur dient gleichzeitig auch als Halte- und Positionierungsstruktur im (Weich-)Gewebe. Andererseits beinhaltet das *in-vivo*-Implantat 1 ein mechanotransduktiv wirkendes elektromagnetisches Induktor- und akustisches Wandlersystem, welches als Schwingkreis direkt gekoppelt und mit Energie- und signaltechnischen Konditionierungen, wie z. B. Kapazitäten, Richterkomponenten, etc. erweitert werden kann. Ex vivo ist ein Wechselfeldgenerator in Gestalt eine Spule platziert.
Im Ausführungsbeispiel gemäß Fig. 1 besteht die elektromagnetische Induktor- und akustische Aktoreinheit innerhalb des *in-vivo*-Implantats 1 aus einem in einem weichmagnetischen, hochpermeablen Rohr mit beispielsweise 3-5 mm Innendurchmesser platzierbaren piezoelektrischen Biegeelement (1,2 nF), vorzugsweise aus Cr/CuNi45- oder Cr/CuNi10-Schichten und mit einer Länge von ca. 15 mm, einer Breite von 2 mm und einer Dicke von ca. 0,5 mm. Wie in Fig. 1 gezeigt, wird der Bieger 3 als Schwinger auf der fixierten Seite kontaktiert und auf der anderen Seite mit einer Klöppelkugel 4 (seismische Masse mit 2-4 mm Durchmesser) versehen. Um das Klöppel-/Schwingrohr 5 als Spulenträger und Kern wird eine Spule 6, 15 mm lang, 0,05 - 0,1 mm Durchmesser Kupferdraht, ca. 10 Lagen, 50 Windungen gewickelt und über elektrische Zwischenglieder mit dem Bieger 3 direkt verbunden. Der Durchmesser der seismischen Masse am Ende des Biegeelementes sollte nicht unter 50 % des Rohrdurchmessers liegen. Im aktiven Modus kann der Bieger 3 als Mechanotransduktionsquelle in der Ausführung gemäß Fig. 1 mit einer Frequenz von 1 - 200 Hz und einer Spannung bis zu 20 - 24 Volt drahtlos betrieben werden und das *in-vivo*-Implantat 1 mechanisch in Schwingung versetzen. Der Bieger 3 ermöglicht z. B. beim Anlegen einer Wechselspannung von 20 Volt Spitze eine Auslenkung von ca. +/-6-7 µm. Die in der Induktoreinheit erzeugte Wechselspannung kann auch als transduktives Signal direkt auf die Oberfläche des *in-vivo*-Implantats 1 parallel oder getrennt steuerbar gelegt werden (Fig. 4).
Prinzipiell sind die rückgewandelten elektrischen Wechselspannungen im inversen Passivbetrieb auch an der Oberfläche des *in-vivo*-Implantats 1 als zusätzliches oder paralleles transduktives Signal applizierbar. Denn im Passivmodus kann der mechanotransduktive Aktor auch als akustischer Energy Harvester benutzt werden, der ebenfalls an die Oberfläche des *in-vivo*-Implantats 1 elektrische Wechselspannungen weiterleiten kann, wenn das implantierte *in-vivo*-Implantat 1 von außen (ex vivo) bspw. über diagnostischen Ultraschall angeregt wird. Dabei wird die applizierte akustische Wellenenergie in elektrische Wechselspannung umgewandelt. Für den Fall eines parallelen aktiven und passiven Betriebs muss der Aktor auch gleichzeitig sensorisch ausgelegt sein. Die seismische Masse müsste in diesem Fall mehr Auslenkung haben, was aber gleichzeitig den aktiven Betrieb nicht vorteilhaft verändert. Für parallele Betriebsarten muss ein optimaler Kompromiss gefunden werden (Fig. 5).
Als Sensor arbeitet das *in-vivo*-Implantat 1 passiv, das heißt energieautark. Dies wird auch als reversiver Betrieb bezeichnet, da der aktorische Betrieb mit externer Energiezufuhr umgekehrt wird. Die mehr oder weniger sensitivere Ausgestaltung der Schnittstellen zwischen Aktor oder Sensor zum umgebenden Formkörper entscheidet darüber, ob das Gesamtsystem primär aktorisch wirkt und damit mittels Energieübertragung von ex vivo zu in vivo akustische bzw. mechanische Impulse einkoppelt oder im reversiven Betrieb energieautark und sensitiv mechanische Belastungsänderungen im Material des umgebenden Formkörpers in elektrische Signale umwandelt und diese elektromagnetisch induktiv nach ex vivo wegsendet.
Das in Fig. 2 dargestellte *in-vivo*-Implantat 1, welches mit einem Endoskopieinstrument mit Steuermimik und Versorgungskanälen fest verbunden ist, ermöglicht die endoskopische Durchleitung und Platzierung des *in-vivo*-Implantats 1 durch das Weich-, Muskel- bis hin zum Organgewebe nach einer minimalinvasiven, laparoskopischen Schnittöffnung an der Haut. Über extern drahtgebundene Energieversorgung über einen endoskopischen Arbeitskanal kann das *in-vivo*-Implantat 1 hilfsweise in Vibration versetzt werden, um den Gewebetransit der Kapsel zu unterstützen (Fig. 6). Nach Erreichen der therapeutisch sinnvollen Endposition kann die endoskopische Verriegelung am *in-vivo*-Implantat 1 gelöst und das Endoskop zurückgeführt werden. Der gesamte Vorgang bzw. der nachfolgende autarke, drahtlose Betrieb des *in-vivo-*Implantats 1 kann per klassischem, medizinisch-diagnostischem Ultraschall (MDUS) 9 überwacht werden (Fig. 7).
In einem weiteren Ausführungsbeispiel kommt im Unterschied zur Ausführung gemäß Fig. 1 kein Bieger 3 sondern stapelbare Multilayer-Piezoaktoren 7 zum Einsatz. Diese Multilayer-Piezoaktoren 7 bestehen aus einer aktiven Piezokeramik, ummantelt von einer isolierenden keramischen Beschichtung. Im Anwendungsfall kommen je nach Dimensionierung des Gesamtsystems je 2- bis 3-fach gestapelte Multilayer-Piezoaktoren 7 mit einer Kantenlänge von 2 bis 3 mm und einer Höhe von 2 mm zum Einsatz. Die aktiven Resonanzfrequenzen sind in allen Fällen größer 600 Hz. Ein Einfluss von magnetischen Wechselfeldern ist ausgeschlossen. Die gewählte Vorspannung würde in diesem Falle zwischen 15 und 30 MPa liegen. Die Schwingungsamplitude würde zwischen 4 bis 7 µm liegen. Die
Schwingungsankopplung würde über die Wand des Resonanz- und / oder Spulenträgerrohres erfolgen. Es können auch entsprechend dem Stand der Technik als Multilayer mehrere Aktoren appliziert werden, die auch in ihrer aktorischen Schwingungsrichtung jeweils parallel zur Leistungsverstärkung oder orthogonal zueinander für eine radiale oder andere Schwingungsrichtung positioniert werden können. Im aktiven Modus kann auch diese Ausführung als Mechanotransduktionsquelle in der o. g. beispielhaften Ausführung mit einer Frequenz von 1 - 200 Hz und einer Spannung bis zu 20 - 24 Volt drahtlos betrieben werden und die immunmodulatorische Plattform mechanisch in Schwingung versetzen. Im Unterschied zu den Ausführungen gemäß Fig. 1 und 8 kommt bei diesem Ausführungsbeispiel erfindungsgemäß ein Rohr-Piezoaktor 8 zum Einsatz. Diese Aktoren bestehen ebenfalls aus einer aktiven Piezokeramik, die auf der Innen- und Außenseite des Rohres kontaktierbar sind, wobei die Stirnflächen des Rohres jeweils isoliert werden. Im Rohr kann ein hochpermeabler Weicheisenkern zur Bündelung des anregenden magnetischen Wechselfeldes angeordnet werden, der gegenüber der Innenseite des Rohr-Piezoaktors 8 isoliert werden kann. Auch die Außenseite des Rohr-Piezoaktors 8 kann isoliert werden, oder die um die Außenseite des Rohr-Piezoaktors 8 gewickelte, induktive Spule 6 ist isolierend ummantelt. Die Anordnung in Fig. 9 integriert ebenso Aktor und Induktor in platzsparender Weise. Der Außendurchmesser eines solchen Rohr-Piezoaktors 8 sollte vorteilhaft zwischen 3,2 und 2,2 mm und der korrespondierende Innendurchmesser zwischen 2,2 und 1 mm betragen. Die Schwingungsamplitude bzw. axiale Kontraktion kann auch hier vergleichbar mit den voranstehenden Ausführungsbeispielen von 5 bis 9 µm reichen. Die aktiven Resonanzfrequenzen sind auch hierbei größer 600 Hz. Ein Einfluss von magnetischen Wechselfeldern ist ausgeschlossen. Im aktiven Modus kann auch diese Ausführung als Mechanotransduktionsquelle in der o. g. beispielhaften Ausführung mit einer Frequenz von 1 - 200 Hz und einer Spannung bis zu 20 - 24 Volt drahtlos betrieben werden und das *in-vivo*-Implantat 1 mechanisch in Schwingung versetzen. Der Vorteil dieser Anordnung liegt in der Definierbarkeit der Schwingungsform.
Für nachfolgende Anwendungsausführungen zum Verfahren zur schonenden Immunmodulation durch Verwendung der Immunmodulatoranordnung sei vorangestellt, dass die Aktorkonditionierung mit Anregungsfrequenzen von 1 - 200 Hz weit unter den vermutlichen Resonanzfrequenzen des Immunmodulators (> 600 Hz) zur Vermeidung von zell- und gewebeschädigenden Nebenwirkungen mit einem Drittel der Risikogrenze bewusst gewählt wurden. Dazu zählen auch die Mikrometeramplituden. Diese entsprechen auch den korrespondierenden Zellgrößen zwischen 15 und 30 µm Durchmesser und Formveränderungs-/anpassungsgeschwindigkeiten von 1,5 bis zu 3 µm/min. Des Weiteren liegt der Vorteil der erfindungsgemäßen topischen Immunmodulation im räumlichen Signalstärkenverlauf. Im Zentrum der Applikation ist der Kraft- bzw. Energieeintrag in der Regel von 2 bis 6 N, jedoch aus Sicherheitsgründen immer < 10 N. Dieser Energieeintrag nimmt mit dem Abstand von der Mechanotransduktionsquelle/ in-vivo-Implantat 1 im Quadrat bzw. reziprok mit dreifacher Potenz ab. Um denselben Energieeintrag transitorisch transkutan bis zum Tumor zu applizieren, beträgt der Energieeintrag an der Applikationsgrenze (Haut) in der Regel > 300 N.

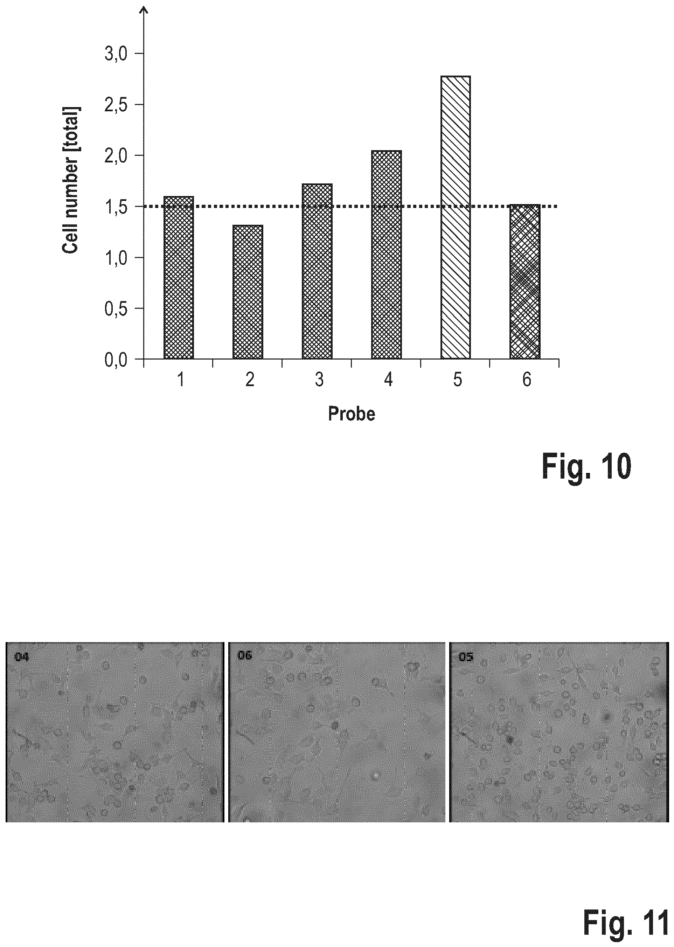

Als Modellsystem wurde im hier angeführten Anwendungsfall die Mikroglia-Zelllinie BV-2 verwendet. Nach einer initialen Adhäsionsphase wurden die Zellen ohne bzw. mit zusätzlichen, proinflammatorischen Stimuli durch LPS und Interferon γ verschiedenen Frequenzen für 24 Stunden für 15 Sekunden in 45 Sekunden Intervallen ausgesetzt. Hierbei wurde bei 16 V und sinusförmigem Kurvenverlauf mit Frequenzen im Bereich 1 bis 200 Hz gearbeitet. In Abhängigkeit von den Frequenzen in Kombination mit oder ohne proinflammatorischen Stimulus zeigte sich in präliminären, ersten Daten ein Einfluss auf die Proliferation, relative Größe und Granularität der Zellen, sowie in der Expression von funktions- und aktivierungsspezifischen Oberflächenmarkern. Die Oberflächenmarker wurden exemplarisch in Hinsicht auf ihre biologische Funktion ausgewählt. Hierbei kann es durch die jeweilige Frequenz sowohl einen induzierenden, wie auch inhibierenden Effekt geben. Der Vorteil dieser Testanordnung sowie der erfindungsgemäßen, topisch wirksamen Immunmodulationsplattform, dem *in-vivo-*Implantat 1, liegt bewusst in beiden Fällen in der Definierbarkeit der Schwingungsform und -stärke, d. h. hier können definierte und reproduzierbare Wirkeffekte induziert werden.
Nachfolgend werden einige Testergebnisse zur Darstellung der Validität der immunmodulatorischen Potenz einer erstmals wirklich topischen Mechanotransduktion aufgeführt.
Gemäß Fig. 10 sind Probe 1-4 (regelmäßig kreuzschraffiert) und 6 (unregelmäßig kreuzschraffiert) mit proinflammatorischem Stimulus (LPS und Interferon γ) stimuliert. Probe 5 (einfach schraffiert) ist ohne proinflammatorischen Stimulus. Proben 1-4 sind verschiedenen Frequenzen im Bereich 1 bis 200 Hz bei 16 V und sinusförmiger Kurve in 45 Sekunden Intervallen für jeweils 15 Sekunden ausgesetzt worden. Die Linie wurde auf Höhe der Probe 6 als Referenz gesetzt. Diese präliminären Daten zeigen, dass nach einer proinflammatorischen Stimulation die Zellproliferation wieder stimuliert oder weiter inhibiert werden kann.

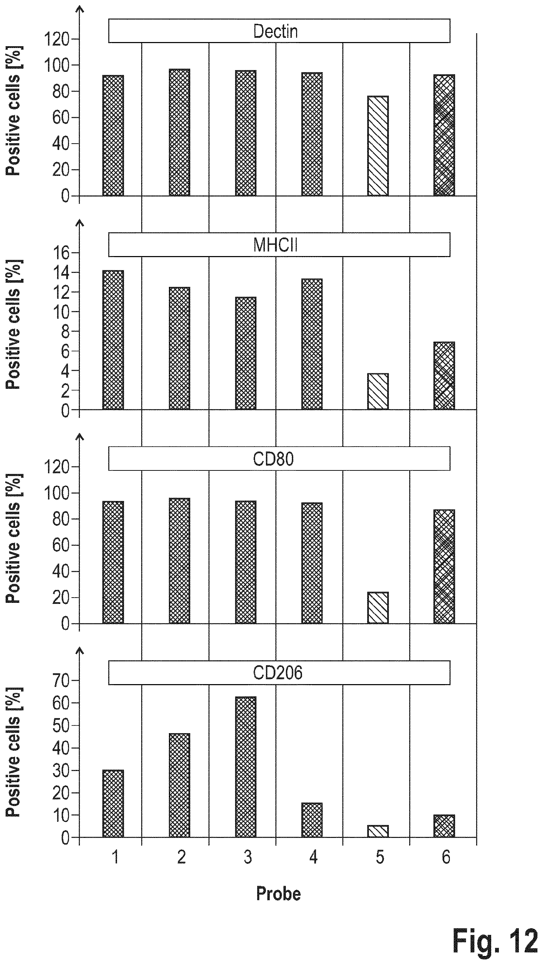

Gemäß Fig. 11 sind Probe 4 und 6 mit proinflammatorischem Stimulus (LPS und Interferon γ). Probe 5 ist hingegen ohne proinflammatorischen Stimulus. Probe 4 ist einer Frequenz im Bereich 1 bis 200 Hz bei 16 V und sinusförmiger Kurve in 45 Sekunden Intervallen für jeweils 15 Sekunden ausgesetzt worden. Diese präliminären Daten zeigen die stärkere Adhäsion der Mikrogliazellen und veränderte Morphologie sowohl durch die proinflammatorische Stimulation, wie auch durch die Mechanotransduktion.
Gemäß Fig. 12 zeigen diese präliminären Daten eine direkte Beeinflussung der Polarisierung der BV-2-Zellen. Proben 1-4 (regelmäßig kreuzschraffiert) und 6 (unregelmäßig kreuzschraffiert) sind mit proinflammatorischem Stimulus (LPS und Interferon γ). Probe 5 (einfach schraffiert) ist ohne proinflammatorischen Stimulus. Proben 1-4 sind verschiedenen Frequenzen im Bereich 1 bis 200 Hz bei 16 V und sinusförmiger Kurve in 45 Sekunden Intervallen für jeweils 15 Sekunden ausgesetzt worden.
Für die Analyse der Polarisierung der BV-2-Zellen wurden verschiedene Oberflächenmarker (MHCII, CD206, Dectin, CD80) gewählt, die bei proinflammatorischer Stimulation (LPS, Interferon gamma) oder einer antiinflammatorischen Stimulation (IL4) hoch reguliert werden.
Die aufgeführten Daten zeigen nochmals ausdrücklich formuliert die immunmodulatorische Potenz sowie die Grenzen einer mechanotransduktiven Modulationsquelle (vgl. Faktorresonanz von Dectin bzw. CD 80).

### Bezugszeichenliste

- 1: *in-vivo*-Implantat
- 2: Endo-/Laparoskopiehalterung
- 3: Bieger
- 4: Klöppelkugel
- 5: Klöppel-/Schwingrohr
- 6: Spule
- 7: Multilayer-Piezoaktor
- 8: Rohr-Piezoaktor
- 9: medizinisch-diagnostischer Ultraschall (MDUS)

## Patentansprüche

1. Immunmodulatoranordnung, bestehend aus einem immunmodulatorischen *in-vivo-*Implantat (1) mit einer Endo-/Laparoskopiehalterung (2) und einem Ex-Vivo-Wechselfeldgenerator, wobei das immunmodulatorische *in-vivo*-Implantat (1) als mechanotransduktiv wirkendes elektromagnetisches Induktor- und akustisches Wandlersystem ausgebildet ist und / oder das immunmodulatorische *in-vivo-*Implantat (1) signalgebende Oberflächenmerkmale aufweist, so dass nach einer endoskopischen/laparoskopischen Platzierung an oder in autologen oder allogenen Zellen und oder Geweben eine mechanotransduktive Immunmodulation und / oder eine aktive und / oder passive Applikation von ein-/angelagerten Signalwirkstoffen zur signaltransduktiven, immunmodulatorischen Anwendung durchführbar ist.

2. Immunmodulatoranordnung nach Anspruch 1, **dadurch gekennzeichnet, dass** das immunmodulatorische *in-vivo*-Implantat (1) eine torpedoähnliche Form aufweist.

3. Immunmodulatoranordnung nach Anspruch 1, **dadurch gekennzeichnet, dass** das *in-vivo-*Implantat (1) eine zur temporären Einlagerung und / oder Anlagerung von Signalwirkstoffen gestaltete Oberfläche aufweist.

4. Immunmodulatoranordnung nach Anspruch 1, **dadurch gekennzeichnet, dass** das *in-vivo*-Implantat (1) eine zur Erzeugung von elektrischen Gleichfeldern und / oder veränderbaren elektrischen Gleichfeldern mit verschiedenen Materialien der elektrochemischen Spannungsreihe ausgestaltete und / oder beschichtete Oberfläche aufweist.

5. Immunmodulatoranordnung nach Anspruch 3, **dadurch gekennzeichnet, dass** die Oberfläche des immunmodulatorischen *in-vivo*-Implantats (1) zur Ein-/Anlagerung der Signalwirkstoffe offenporig ausgebildet ist.

6. Immunmodulatoranordnung nach Anspruch 3 oder 5, **dadurch gekennzeichnet, dass** die Oberfläche des immunmodulatorischen *in-vivo*-Implantats (1) zur Ein-/Anlagerung der Signalwirkstoffe Rillen aufweist.

7. Immunmodulatoranordnung nach Anspruch 1 bis 6, **dadurch gekennzeichnet, dass** das immunmodulatorische *in-vivo*-Implantat (1) einen weichmagnetischen, hochpermeablen Rohrabschnitt aufweist, in dem ein piezoelektrisches Biegeelement angeordnet ist.

8. Immunmodulatoranordnung nach Anspruch 7, **dadurch gekennzeichnet, dass** mindestens der Rohrabschnitt aus Cr/CuNi45 oder Cr/CuNi10 Schichten ausgeführt ist.

9. Immunmodulatoranordnung nach Anspruch 1, **dadurch gekennzeichnet, dass** das immunmodulatorische *in-vivo*-Implantat (1) stapelbare Multilayer-Piezoaktoren (7) aufweist.

10. Immunmodulatoranordnung nach Anspruch 9, **dadurch gekennzeichnet, dass** die als Multilayer konfektionierten Piezoaktoren (7) aus einer aktiven Piezokeramik, ummantelt von einer elektrisch und oder thermisch isolierenden keramischen und oder metallischen Beschichtung, bestehen.

11. Immunmodulatoranordnung nach Anspruch 1, **dadurch gekennzeichnet, dass** das immunmodulatorische *in-*vivo*-*Implantat (1) einen aus einer aktiven Piezokeramik bestehenden Rohr- und oder Biege- und oder Scheiben- Piezoaktor (8) aufweist.

12. Immunmodulatoranordnung nach Anspruch 1 bis 11, **dadurch gekennzeichnet, dass** der Wechselfeldgenerator eine oder mehrere räumlich angeordnete Spulen sind und oder mindestens einen rotierenden Permanentmagnet enthält.

13. Verfahren zur topischen Einkopplung von in Betrag und Richtung wechselnder Energie nach Platzierung der Immunmodulatoranordnung nach einem der Ansprüche 1 bis 12 an oder in autologen oder allogenen Zellen und oder Geweben, **dadurch gekennzeichnet, dass** kinetische Impulse als mechanische Ortsveränderungen und in Richtung und Betrag wechselnd an einem Wirkort gegenüber dem *in-vivo-*Implantat (1) bzw. über die Oberfläche des *in-vivo*-Implantats (1) selbst appliziert werden, dass elektrische Felder in Richtung und Betrag wechselnd an und / oder über die Oberfläche des *in-vivo*-Implantats (1) in die Umgebung eingekoppelt werden und Bindungsenergien an der Oberfläche des *in-vivo*-Implantats (1) beeinflussen und damit chemische Bindungen zu lösen helfen, dass mit der direkten, unmittelbaren Einbringung kinetischer Energie Lösungs- und Konzentrationsänderungen von chemischen Verbindungen an der Oberfläche des *in-vivo*-Implantats (1) hervorgerufen werden können, um Beschichtungen in Medien sowie auch clickchemische Bindungen an der Oberfläche des *in-vivo*-Implantats (1) lösen zu können.
